# EUROPEAN PATENT APPLICATION

(11) **EP 0 693 298 A1**
(43) Date of publication of application: **24.01.1996**
(21) Application number: 95111273.9
(22) Date of filing: 18.07.1995
(51) Int. Cl.: A61M 1/34, A61M 1/16

(54) **Perfected dialysis apparatus**

(30) Priority: 18.07.1994 IT BO940335
(71) Applicant: BELLCO S.p.A., I-41037 Mirandola(Modena) (IT)
(72) Inventor: Cianciavicchia, Domenico, I-64040 Cavuccio (IT); Morselli, Massimo, I-41038 S. Felice sul Panaro (IT)
(74) Representative: Jorio, Paolo

(57) **Abstract**

An apparatus hemodiafiltration (1) presenting a haemofilter (2); a first tube (3) for feeding blood from an artery (4) to the haemofilter (2); a haemodialyzer (6) connected by a second tube (7) to the haemofilter (2); a third tube (8) for feeding blood from the haemodialyzer (6) to a vein (9); a fourth tube (11) for draining the ultrafiltered fluid withdrawn from the blood by the haemofilter (2); and a dialysis machine (13) for preparing the dialysis solution whereby toxic substances are removed from the blood. The apparatus (1) also presents means (17) for detecting the quantity of saline ions in the blood fed along the first tube (3); means (12) for detecting the quantity of saline ions in the blood fed along the fourth tube (11); and means (21) for processing the above findings to define a value indicating the percentage quantity of red and white cells in the blood.

## Description

The present invention relates to a perfected dialysis apparatus.

As is known, one of the parameters to be controlled in patients subjected to dialysis treatment is the percentage quantity of red and white cells in the blood.

It is an object of the present invention to provide a perfected dialysis apparatus featuring a device for controlling, during treatment, the percentage quantity of red and white cells in the blood.

It is a further object of the present invention to provide a method of determining, during treatment, the percentage quantity of red and white cells in the blood.

According to the present invention, there is provided a perfected dialysis apparatus comprising:
a haemofilter;
a first tube for feeding the blood to be purified from an artery of the patient to the inlet of said haemofilter;
a haemodialyzer;
a second tube for feeding the blood from said haemofilter to the inlet of said haemodialyzer;
a third tube for feeding the purified blood from said haemodialyzer to a vein of the patient;
a fourth tube for draining the ultrafiltered fluid withdrawn from the blood by said haemofilter; and
a dialysis machine for preparing the dialysis solution whereby the toxic substances in the blood are removed by said haemodialyzer;
characterized in that it comprises:
first means, installed along said first tube, for detecting the percentage quantity of saline ions in the blood fed along the first tube;
second means, installed along said fourth tube, for detecting the percentage quantity of saline ions in the blood fed along the fourth tube; and
means for processing the above findings, to define a parameter indicating the percentage quantity of red and white cells in the blood.

According to the present invention, there is also provided a method of determining the percentage quantity of red and white blood cells in a dialysis apparatus comprising:
a haemofilter;
a first tube for feeding the blood to be purified from an artery of the patient to the inlet of said haemofilter;
a haemodialyzer;
a second tube for feeding the blood from said haemofilter to the inlet of said haemodialyzer;
a third tube for feeding the purified blood from said haemodialyzer to a vein of the patient;
a fourth tube for draining the ultrafiltered fluid withdrawn from the blood by said haemofilter; and
a dialysis machine for preparing the dialysis solution whereby the toxic substances in the blood are removed by said haemodialyzer;
characterized in that it comprises:
a first step in which means detect the percentage quantity of saline ions in the blood fed along said first tube;
a second step in which means detect the percentage quantity of saline ions in the ultrafiltered fluid fed along said fourth tube; and
a third step in which means process signals corresponding to the above findings; the outcome of said processing indicating the percentage quantity of red and white cells in the blood.

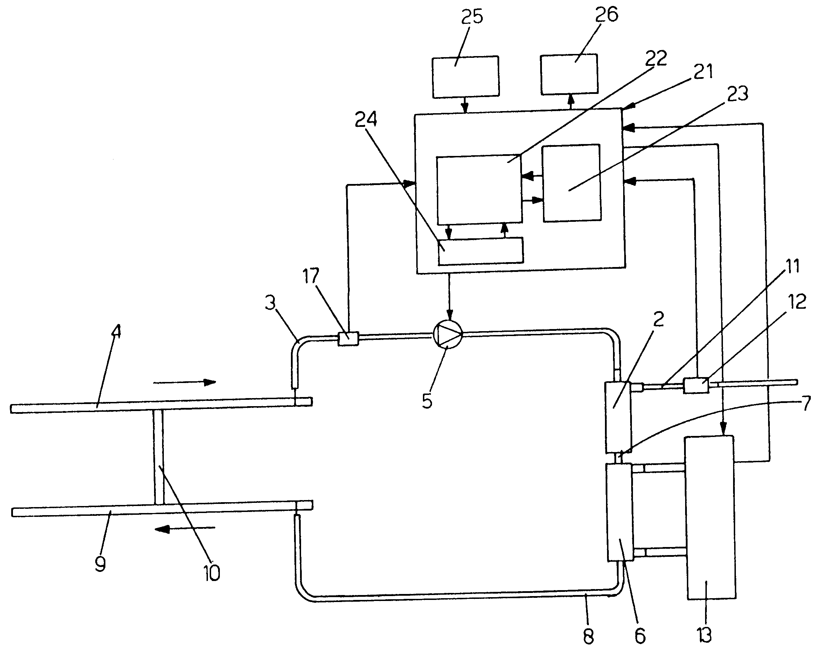

A preferred, non-limiting embodiment of the present invention will be described by way of example with reference to the accompanying drawing, which shows a block diagram of a perfected dialysis apparatus indicated as a whole by 1.

Apparatus 1 comprises:
a known haemofilter 2;
a tube 3 for feeding the blood to be purified from an artery 4 (shown schematically) of the patient to the inlet of haemofilter 2;
a preferably peristaltic pump 5 along tube 3;
a known haemodialyzer 6;
a tube 7 for feeding the blood from haemofilter 2 to the inlet of haemodialyzer 6;
a tube 8 for feeding the purified blood from haemodialyzer 6 to a vein 9 of the patient, said vein 9 communicating hydraulically with artery 4 via a fistula 10;
a tube 11 for feeding to a drain (not shown) the ultrafiltered fluid withdrawn from the blood by haemofilter 2;
a conductivity sensor 12 along tube 11;
a known dialysis machine 13 for preparing the dialysis solution whereby the toxic substances in the blood are removed by haemodialyzer 6;
a conductivity sensor 17 along tube 3; and
an electronic control unit 21 for controlling pump 5 and machine 13, and to which sensors 12 and 17 are connected.

Control unit 21 comprises a central data processing unit 22, a memory block 23, and a timing block 24, and is connected to a keyboard 25 and a display 26.

As is known, blood substantially comprises red cells, white cells, platelets, and plasma which in turn substantially comprises water, protein and saline ions; and a conductivity sensor generates an electric signal proportional to the electric conductivity of the fluid under examination. If the fluid being examined is blood, its electric conductivity is proportional to the percentage quantity of saline ions contained in it, so that a conductivity sensor installed along an external blood circuit generates an electric signal proportional to the percentage quantity of saline ions in the blood.

It is also known that the ultrafiltered fluid is perfectly equivalent to plasma with no protein, so that a conductivity sensor installed along the ultrafiltered fluid circuit generates an electric signal proportional to the percentage quantity of saline ions in the blood from which the ultrafiltered fluid is derived. In other words, sensor 17 determines directly the percentage quantity of saline ions in the blood fed along tube 3, and sensor 12 determines indirectly the percentage quantity of saline ions in the blood fed along tube 11. A comparison of the findings of sensors 17 and 12 therefore gives a value indicating the percentage quantity of red and white cells in the blood.

The method of determining the percentage quantity of red and white cells in the blood substantially comprises:
a first step to detect the percentage quantity of saline ions in the blood fed along tube 3;
a second step to detect the percentage quantity of saline ions in the ultrafiltered fluid fed along tube 11; and
a third step in which signals corresponding to the above findings are processed; the outcome of said processing indicating the percentage quantity of red and white cells in the blood.

In actual use, processing of the signals may comprise, firstly, a straightforward comparison to define a value, and, secondly, a reading of the value in a table stored in memory block 23, defined on the basis of laboratory tests, and containing, for each comparison value, a parameter indicating the percentage quantity of red and white cells in the blood.

In any case, the processing results may be memorized in block 23 for retrieval prior to the next dialysis treatment, thus enabling the percentage quantity of red and white cells in the blood to be controlled repeatedly during the same treatment, and from one treatment to the next. The processing data and results may also be displayed on display 26.

The advantages of the present invention will be clear from the foregoing description.

In particular, it provides for an apparatus capable of controlling, during treatment, the percentage quantity of red and white cells in the blood.

Clearly, changes may be made to apparatus 1 as described and illustrated herein without, however, departing from the scope of the present invention.

In particular, apparatus 1 may present a further pump along tube 8; and sensors 12, 17 may be either invasive or noninvasive.

## Claims

**1)** A perfected dialysis apparatus comprising:
a haemofilter (2);
a first tube (3) for feeding the blood to be purified from an artery (4) of the patient to the inlet of said haemofilter (2);
a haemodialyzer (6);
a second tube (7) for feeding the blood from said haemofilter (2) to the inlet of said haemodialyzer (6);
a third tube (8) for feeding the purified blood from said haemodialyzer (6) to a vein (9) of the patient;
a fourth tube (11) for draining the ultrafiltered fluid withdrawn from the blood by said haemofilter (2); and
a dialysis machine (13) for preparing the dialysis solution whereby the toxic substances in the blood are removed by said haemodialyzer (6);
characterized in that it comprises:
first means (17), installed along said first tube (3), for detecting the percentage quantity of saline ions in the blood fed along the first tube (3);
second means (12), installed along said fourth tube (11), for detecting the percentage quantity of saline ions in the blood fed along the fourth tube (11); and
means (21) for processing the above findings, to define a parameter indicating the percentage quantity of red and white cells in the blood.

**2)** An apparatus as claimed in Claim 1, characterized in that said first (17) and second (12) detecting means comprise respective electric conductivity sensors.

**3)** An apparatus as claimed in Claim 2, characterized in that said processing means comprise an electronic control unit (21) in turn comprising a central data processing unit (22), a memory block (23), and a timing block (24); said control unit (21) also being connecting to a data programming keyboard (25) and a programmed data and processing result display (26).

**4)** A method of determining the percentage quantity of red and white blood cells in a dialysis apparatus comprising:
a haemofilter (2);
a first tube (3) for feeding the blood to be purified from an artery (4) of the patient to the inlet of said haemofilter (2);
a haemodialyzer (6);
a second tube (7) for feeding the blood from said haemofilter (2) to the inlet of said haemodialyzer (6);
a third tube (8) for feeding the purified blood from said haemodialyzer (6) to a vein (9) of the patient;
a fourth tube (11) for draining the ultrafiltered fluid withdrawn from the blood by said haemofilter (2); and
a dialysis machine (13) for preparing the dialysis solution whereby the toxic substances in the blood are removed by said haemodialyzer (6);
characterized in that it comprises:
a first step in which means (17) detect the percentage quantity of saline ions in the blood fed along said first tube (3);
a second step in which means (12) detect the percentage quantity of saline ions in the ultrafiltered fluid fed along said fourth tube (11); and
a third step in which means (21) process signals corresponding to the above findings; the outcome of said processing indicating the percentage quantity of red and white cells in the blood.
